# EUROPEAN PATENT SPECIFICATION

(11) **EP 4 096 428 B1**
(45) Date of publication and mention of the grant of the patent: **11.10.2023**
(21) Application number: 21702016.3
(22) Date of filing: 26.01.2021
(51) Int. Cl.: A23K 20/111, A23K 20/137, A23K 50/10, A61K 31/48

(54) **VETERINARY ANTI-PROLACTIN COMPOSITION FOR RUMINANTS USED BY INTRAMAMMARY ADMINISTRATION**
VETERINÄRMEDIZINISCHE ANTI-PROLAKTIN-ZUSAMMENSETZUNG FÜR WIEDERKÄUER ZUR INTRAMAMMÄREN VERABREICHUNG
COMPOSITION ANTI-PROLACTINE VÉTÉRINAIRE POUR RUMINANTS UTILISÉE PAR ADMINISTRATION INTRAMAMMAIRE

(30) Priority: 27.01.2020 EP 20305066
(43) Date of publication of application: 07.12.2022
(73) Proprietor: Ceva Santé Animale, 33500 Libourne (FR)
(72) Inventor: BERTAIM, Thierry, 33500 LIBOURNE (FR); DEFLANDRE, Audrey, 33500 LIBOURNE (FR)
(74) Representative: Cabinet Becker et Associés
(86) International application number: PCT/EP2021/051766
(87) International publication number: WO 2021/151897

(56) References cited:
- US-A1- 2011 245 261
- US-A1- 2013 203 762
- US-A1- 2019 358 154
- BOUTINAUD M ET AL: "Cabergoline inhibits prolactin secretion and accelerates involution in dairy cows after dry-off", JOURNAL OF DAIRY SCIENCE, vol. 99, no. 7, 2015, pages 5707-5718, XP029602061, ISSN: 0022-0302, DOI: 10.3168/JDS.2015-10782
- P. LACASSE ET AL: "Review: Inhibition of prolactin as a management tool in dairy husbandry", ANIMAL, vol. 13, no. S1, 1 July 2019 (2019-07-01), pages s35-s41, XP055677643, GB ISSN: 1751-7311, DOI: 10.1017/S1751731118003312
- Anonymous: "Velactis: Pending EC decision | European Medicines Agency", , 13 September 2019 (2019-09-13), XP055677718, Retrieved from the Internet: URL:https://www.ema.europa.eu/en/medicines /veterinary/summaries-opinion/velactis [retrieved on 2020-03-19]

## Description

The present invention relates to a veterinary composition and to a kit for reducing milk production in dairy ruminants according to a more efficient and safer way, especially at the start of the dry period (i.e. abrupt milking cessation). It consists in administering said composition by intramammary (IMM) route at the time of the dry-off, i.e. after the last milking.

In dairy ruminants, lactation generally lasts for 5 to 20 months. In dairy cows it is most often 10 months. After this lactation period, milking is stopped, generally abruptly, and the dairy ruminant produces no more milk until calving, when the subsequent lactation begins. This period between the cessation of milking and the subsequent lactation, which is generally at parturition (or calving with respect to cows), known as the "dry period", is generally set at about 2 months (i.e. 45-70 days) in dairy cows.

Although a dry period is necessary to ensure optimal milk production during the subsequent lactation, this dry period necessitates several dietary changes and transitions to adapt to the physiological needs of an animal that is no longer producing milk. These dietary changes cause numerous physiological disruptions and stresses in ruminants. This dry period leads to a metabolism in dairy ruminants that is very different from that observed during lactation. The transition from lactating to non-lactating status favorizes the outcome of transitory disruption of the homeostasis of dairy cows.

During the dry period, and more particularly within the first 24 hours following the dry-off, milk synthesis in the mammary gland continues, especially with lactating ruminants that have a high level of milk production. Consequently, needs for calcium transfer from blood to the udder milk persist during these first 24 hours. In addition, at the dry period implementation, the breeding farmers decrease very often the food ration which further decreases the calcium supply and its level in blood circulation. Moreover, the antiprolactinic products, as to help the drying off, have also a known effect which decreases the intestinal absorption of the calcium coming from the food, when generally administered by parenteral route as intramuscular (IM). All this contributes to increase the risk of hypocalcemia at the time of lactation drying off.

At drying off, some dairy cows receiving the veterinary composition by intramuscular route after the last milking, may thus experience hypocalcemia within 24 hours after treatment administration. Hypocalcemia in some cases can cause the recumbency of the cow which requires appropriate therapy given by the veterinarian. A way to know whether dairy cows are experiencing hypocalcemia is to analyze blood for the concentration of calcium within the first 1 to 2 days after the last milking. Dairy cows with blood calcium concentrations at or below 8.8 mg/dl (2.2 mmol/l) but not showing clinical signs are considered subclinically hypocalcemic. This latter status is also considered as deleterious for the cow health, which can favorize further the outcomes of different metabolic disorders for the cow.

Meanwhile, there is a need for a treatment that reduces milk production efficiently so that the mammary gland stops quickly its milk synthesis, without excessive udder engorgement due to milk accumulation and stasis. This will help to prevent from negative impacts on dairy cow health. Indeed, a less udder engorgement is associated with a reduction in milk leakages which is a high-risk factor for new intramammary infections in dairy cow. It also reduces pain and discomfort of the cow during the day following drying-off. At last, it enhances the mammary gland involution during the dry period, a key physiological process for the health and regeneration of the gland for the next lactation.

In veterinary medicine, prolactin inhibitor-based compositions such as Galastop^{®} (cabergoline, Ceva Santé Animale) can be administered as treatments for lactation during false pregnancies in bitches. They are also prescribed in order to halt milk production in ruminants. WO2010040765 in the name of the Applicant describes the administration of anti-prolactin dopamine receptor agonist compounds, such as cabergoline, to ruminants during gestation in order to facilitate lactation dry-off and to promote mammary involution, especially by intramuscular administration. However, such intramuscular administration of cabergoline can possibly induce hypocalcemia in dairy cows, in particular during the first 24 hours post administration following the dry-off, for the reasons as explained in the previous sections.

The Applicant has now discovered, surprisingly, that the administration of a veterinary composition comprising cabergoline to ruminants via intramammary route at the time the dry off is implemented can reduce milk production more efficiently. Surprisingly, intramammary administration of the veterinary compositions according to the present invention improves bioavailability of cabergoline in ruminants allowing thereby more satisfactory efficacy results in terms of reduction of milk production and/or reduction of udder engorgement. In addition, and surprisingly, it has also been found that intramammary administration of cabergoline allows prevention and/or reduction of hypocalcemia in comparison with cabergoline used by intramuscular route, especially within the first 24 hours after milking cessation.

### Summary of the invention

The present invention relates to veterinary compositions comprising cabergoline for use in a treatment that reduces lactation or milk production of a dairy ruminant, wherein said composition is administered via intramammary route to dairy ruminants, preferably at the time of the implementation of the dry period, i.e. at the time of the dry-off.

According to the invention, said compositions are administered by intramammary route in effective therapeutic amounts, preferably at the time of the implementation of the dry period of dairy ruminants, in order to induce a reduction of milk production in dairy ruminants.

The compositions are administered by intramammary route to dairy ruminants in a single treatment and/or in repeated treatments, preferably in a single treatment.

The intramammary administration of veterinary compositions according to the invention moreover does not affect the milk-producing ability and/or milk quality of the ruminant during the subsequent lactation.

The present invention also relates to kits for reducing milk production of dairy ruminants comprising a veterinary composition as defined above, and optionally means for intramammary administration of cabergoline.

### Description of the Figures

**Figures 1****:** Milk production (L) at morning milking from D2 to D9 - Cabergoline treatment at D4 evening milking (arrow). Mean of the group.
**Figures 2****:** Inter-Teat Perimeter (cm) from treatment day D0 to D9 post-treatment. Means per group. (control: top curve; treated cows: bottom curve)
**Figures 3****:** Delta Inter-Teat Perimeter (ITP) (cm) from treatment day D0 to D9 (+216 h) post-treatment. Means per group. (control: top curve; treated cows: bottom curve)
**Figure 4****:** mean and sd plasma concentration (pg/mL) - time (hour) profiles of intra-mammary administration of cabergoline
**Figure 5****:** mean and sd plasma concentration (pg/mL) - time (hour) profiles of intramuscular administration of cabergoline (comparative study)
**Figures 6A and 6B****:** mean(sd) blood Ca concentration (mmol/L) (graph A: control "ctrl" cows versus cabergoline IMM treated "cab IMM" cows) and difference relative to baseline (0h) pre-treatment with respect to time (hour) (graph B: control "ctrl" cows versus cabergoline IMM treated "cab IMM" cows).
**Figure 7****:** comparison of mean blood Ca concentrations according time, between different studies: IMM study (top curve) and IM studies (2 bottom curves, named IM studies 1 and 2).

### Detailed description of the invention

The phrases "dry period" includes the period of time between last milking of the lactation period and the subsequent lactation of the subsequent lactation period, which starts generally at parturition. The dry period generally lasts about 2 months, more specifically from 56 to 70 days in dairy cows.

In the context of the invention, the terms "last milking", "dry-off' or "milking cessation" can be used interchangeably. They correspond to the time of the implementation of the dry period.

The present invention relates to veterinary compositions comprising cabergoline for use in a treatment that reduces milk production, wherein said composition is administered via intramammary route to dairy ruminants, preferably at the time of the implementation of the dry period.

The present invention also relates to a veterinary composition comprising cabergoline administered via intramammary route in a single treatment and/or in repeated treatments, preferably at the time of the implementation of the dry period, for use to reduce milk production.

The compositions used according to the invention also allow to reduce milk production, induce mammary involution in ruminants avoiding thereby intra-mammary infections or inflammations.

Moreover, it is interesting to note that such intramammary administration of the compositions of the invention allows to improve bioavailability of cabergoline in ruminants, rendering possible the administration of a lower dose of cabergoline when compared to a intramuscular administration while obtaining similar results and/or efficacy or rendering possible the administration of a dose of cabergoline similar to a dose of cabergoline by intramuscular administration while obtaining higher results and/or efficacy.

Furthermore, the inventors surprisingly noticed that the veterinary composition comprising cabergoline administered via intramammary route also enables to prevent or reduce hypocalcemia in ruminants during the dry period, especially within the first 24 hours after milking cessation in comparison with cabergoline when administered by IM route.

When said compositions are intramammary administered to ruminants at the time of the implementation of the dry period, they make it possible to maintain a blood calcium concentration higher than or equal to 2.1 mmol/l or preferably to 2.2 mmol/l in ruminants, even without any specific diet and more specifically even without any diet regimen aiming at maintaining a satisfactory blood calcium concentration in ruminants (such as diets with adequate amounts of magnesium and balance these diets to provide a DCAD). The compositions used according to the invention thus prevent from hypocalcemia or diminish the risk of developing hypocalcemia, especially within the first 24 hours after dry-off, i.e. when the risk of developing hypocalcemia is high when using cabergoline by IM route.

Disclosed herein, but not claimed, is a method of reducing milk production of dairy ruminants, comprising an intramammary administration according to an effective therapeutic regimen of the veterinary compositions to ruminants, preferably at the time of the implementation of the dry period.

Preferably, said veterinary compositions are administered to dairy cows.

The veterinary compositions for use according to the invention are intramammary administered, more specifically, at the time of the dry-off, after the last milking. In a particular embodiment, the veterinary compositions are intramammary administered, preferably after the last milking and more preferably within the first 5, the first 4 or the first 2 hours after the last milking, preferably within the first 30 minutes (such as 15-20 minutes) after the last milking.

The veterinary compositions according to the present invention are attractive to the farmers as their use is simple to implement allowing a rapid cessation of milk production in mammary glands, without inducing udder engorgement. They can also maintain a satisfactory blood calcium concentration, without worrying about a specific blood calcium concentration increasing-regimen in comparison when cabergoline is used by IM route. The treatment of the invention results in a simplified herd management, and savings in terms of disease treatment costs when lactation is resumed. The administration of the compositions according to the present invention thus enables better management of dietary programs during the dry period.

Cabergoline, whose chemical name is N-[3-(dimethylamino)propyl]-N-[(ethylamino)carbonyl]-6-(2-propenyl)-8g-ergoline-8-carboxamide, is an anti-prolactin agonist compound specific for D2 dopamine receptors. It is in particular described in the patent US 4,526,892. Its chemical structural formula is the following:

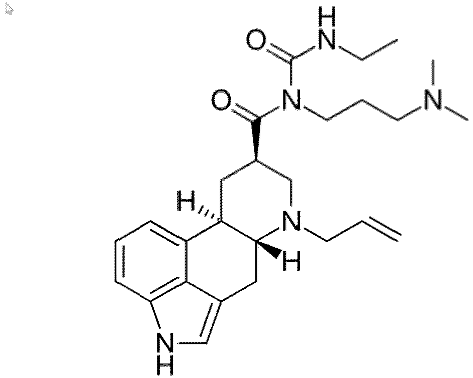

Cabergoline is the active ingredient in human drugs marketed under the names Dostinex^{®} and Cabaser^{®}. Also, it is the basic active ingredient in veterinary compositions marketed under the name Galastop^{®} for bitches prone to lactations of false pregnancy. Neither of these cabergoline-based compositions, Dostinex^{®} or Galastop^{®}, is administered by intramammary route.

The effective amounts or therapeutic doses of cabergoline to be administered according to the invention are likely to vary according to the ruminants to be treated. The dosages, also called therapeutic regimens, can easily be determined by systematic tests on the basis of the examples below and are within the ability of persons skilled in the art. According to a particular embodiment of the invention, the effective therapeutic doses according to the present invention are between 1 and 50 µg/kg, or between 5 and 50 µg/kg, preferably about 5 to 11 µg/kg, and more preferably about 6 to 9 µg/kg.

More preferably, the amount of cabergoline which is intramammary administered is from 0.25 to 7 mg, preferably from 0.3 to 6 mg, and more specifically 1-6 mg, per dairy ruminant.

More preferably, the amount of cabergoline which is intramammary administered is from 2 to 7 mg, preferably from 3 to 6 mg, and more specifically 5-6 mg, per dairy cow.

According to a particular embodiment, the amount of cabergoline which is intramammary administered is from 2 to 4 mg, preferably from 3 to 3.5 mg, per dairy ruminant, and preferably per dairy cow.

According to the invention, the term "ruminants" refers to herbivorous mammals such as, for example, bovines, ovines, caprines, camelids or bovids. The compositions according to the invention are administered to milk-producing (or dairy) ruminant mammals, preferably such as dairy cows, sheeps and goats, and more preferably dairy cows.

Advantageously, the veterinary compositions of the present invention can be administered in association with standard treatments for treating and/or preventing intramammary inflammations and intramammary infections (such as mastitis) of ruminants. Examples of standard care or prophylactic compositions of mastitis are local disinfectants for udders, antibiotics such as penicillins, cephalosporines, gentamycin or teat sealants.

As mentioned before, hypocalcemia is caused by a temporary blood calcium deficiency which usually occurs around the time of calving, when lactation is resumed, but can occur at another period, especially within the first 48 hours, and more frequently within the first 24 hours, following the dry-off of the milk-producing ruminant after administration of cabergoline by IM route. According to a particular embodiment of the invention, preventing and/or reducing hypocalcemia in a dairy ruminant refers to a treatment where the ruminant presents or attains a satisfactory blood calcium concentration which is higher than or equal to 2.1 mmol/l, preferably 2.2 mmol/L.

The veterinary compositions are administered by intramammary routes. To this end, the veterinary composition is administered in one or more quarters of the mammary glands by introduction of the composition via infusion, implantation, injection, or a combination thereof. The administration is generally carried out via the teat canal by intramammary infusion. Implantation may be in the form of a solid, paste, gel, or suspension. Infusion or injection described herein can be by bolus injection or continuous infusion. Formulations for injection may be presented in unit dosage form, e.g., in ampoules or in multidose containers with optionally, an added preservative. The compositions may be suspensions, solutions or emulsions in oily or aqueous vehicles.

Preferably, the composition is administered to one or more, preferably all, quarters of the mammary gland of the ruminant. For instance, in a dairy cow, it is administered to 1, 2, 3 or 4 quarters, preferably to all 4 quarters of the udder.

According to the formulations of the compositions used, they can further comprise ingredients conventionally used in pharmacy for the preparation of liquid or solid formulations for intramammary administration.

Also, the compositions according to the invention can comprise according to the type of formulations, a solvent, a glidant, a lubricant and any excipient of suitable mass, such as lactose, cellulose or starches. Stearic acid, magnesium stearate, L-leucine or, for example, glycerol tribehenate can be used as a lubricant. As disintegrating agent, sodium carboxymethylamidone, cross-linked sodium carboxymethyl cellulose or cross-linked polyvinylpyrrolidone can be used. As glidants, pure silica or colloidal silicon dioxide can be used. Solid oral forms can be in the form of tablets covered with a coating.

Injectable preparations are prepared by mixing effective therapeutic amounts of at least one anti-prolactin compound as described above with a solvent, a pH regulator, a buffer agent, a suspending agent, a solubilizing agent, a stabilizer, a tonicity agent and/or a preservative, and by transforming the mixture into a subcutaneous or intramuscular injection according to a conventional method. As solvent, mention may be made of dimethylsulfoxide (DMSO), oily solvents such as medium-chain triglycerides, or a mixture of capric acid, caprylic acid and triglycerides such as that marketed under the name Mygliol^{®}812. As needed, the injectable preparations can be lyophilized according to a conventional method. Examples of suspending agents include methyl cellulose, polysorbate 80, hydroxyethylcellulose, xanthan gum, sodium carboxymethylcellulose and polyethoxylated sorbitan monolaurate. Examples of solubilizing agents include polyoxyethylene hardened castor oil, polysorbate 80, nicotinamide, polyethoxylated sorbitan monolaurate, macrogol and castor oil fatty acid ethyl ester. Furthermore, stabilizers include sodium sulfite, sodium metal sulfite and ether, while preservatives include methyl p-hydroxybenzoate, ethyl p-hydroxybenzoate, sorbic acid, benzyl alcohol, phenol, cresol and chlorocresol. An example of a tonicity agent is mannitol.

The present invention further relates to a kit for veterinary use, more particularly for use in reducing milk production of dairy ruminants. The kits according to the present invention include at least one compartment for an optionally sterile packaging including an effective therapeutic amount of cabergoline as described above, for intramammary administration to ruminants. The kit can also include the means enabling the administration of the composition by intramammary route and/or instructions relating to the mode and time of administration of the veterinary compositions or drugs according to the invention.

All embodiments described above for the composition and use of said composition also apply to the kit and to the method of the present invention.

Described herein below are examples. These examples are illustrative and in no way limiting.

### EXAMPLES

### Example 1: Cabergoline effect by intramammary (IMM) route on milk production in dairy cow (milking continuation context)

The cabergoline composition is a solution comprising cabergoline with dimethyl sulfoxide and medium-chain triglycerides, as excipients. It corresponds to the product called Velactis^{™} (cabergoline 1.12mg/mL).

5 Prim'holstein lactating cows were included in the study, with the following characteristics:
- Multiparous: parity 2 to 4
- Daily milk yield (L): 17.4 +/- 2.8
- Days in milk (d): 251 +/-51
- Body weight (kg): 686+/-29

Cabergoline was administered as an oily solution as described above, with an intramammary cannula inserted into the teat canal and fitted to the tip of a syringe containing the solution. A volume of 10 mL was directly deposited into the udder cistern of each quarter. Velactis^{™} (cabergoline 1.12mg/mL) was diluted with its excipient, just before administration, in accordance with the tested dose.

Cows were milked twice a day, morning and evening, at around 07am and 05 pm respectively.

Milk production (morning milking) was measured during the days before (baseline) and after treatment. At D0 study, 3/5 cows were treated with the excipient only, and showed that the excipient has no effect on milk production.

The 5 cows were treated with a dose 1.34 mg/ quarter in all 4 quarters (total dose 5.4mg/cow, corresponding to ~7.5µg/kg when expressed according to kg body weight, after evening milking (D4), i.e. 15-20 minutes after the end of milking.

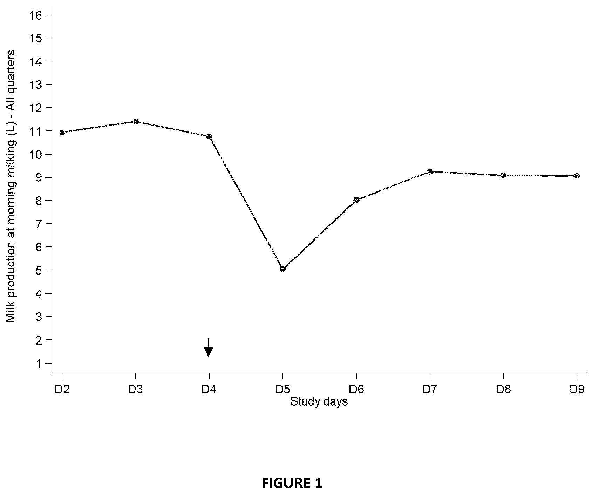

All the 5 cows exhibited a sharp drop of milk production of minus 6 L in mean at the next morning milking (D5), representing a mean decrease of -54% (sd=10, min-38%, max-62%) relative to milk production baseline value (D2 to D4). Then, the milk production progressively increased back over the following days (around -15% of the baseline, 3 days after treatment), see Figure 1.

These results obtained by IMM route compared favorably with the results obtained by IM (intramuscular) route obtained from another study performed previously. In this previous study, different IM doses were tested, in order to establish the dose-effect relationship of cabergoline on milk production reduction. The obtained results are the following:

**Table 1 (comparative results)**

| *Study (Velactis^{™}, IM route oily solution 1.12mg*/*mL)* | | | |
|---|---|---|---|
| Doses tested (µg/kg) (N/group = 6) | Milk production reduction (%) | | |
| | Mean | (sd) | [min , max] |
| 0 | -4 | (6) | [+2 , -16] |
| 1.33 | - 19 | (36) | [+26 , -77] |
| 4 | - 35 | (31) | [+2 , -83] |
| 12 | - 53 | (34) | [+9 , -87] |

Extrapolation of the dose-effect relationship, with an IM dose of 7.5µg/kg gave a predicted effect on milk production reduction of -43%. This result was confirmed by another study (field-controlled study, n=106) where decrease of milk production was found at around -40% for treated against negative controlled cows.

Thus, it is concluded that cabergoline administered by IMM route at a dose of 1.34mg/quarter in all four quarters exhibits a better effect on milk production reduction compared to similar total dose of 5.6mg/cow (i.e. ~7.5µg/kg) administered by IM route which is the registered dose for Velactis^{™}. This better effect was similar with the effect observed at a higher IM dose of cabergoline (12µg/kg). However, this high dose (12µg/kg) by IM route is not actually used in ruminants. This conclusion is also supported by pharmacokinetics results obtained in a study detailed below, showing the effect proportionality on milk production reduction related with the systemic cabergoline exposure (see example 2.2). Moreover, the milk production reduction obtained by IMM route appeared to be more reliable and reproducible, as sd, min and max values were less dispersed compared to the ones with IM route.

### Example 2. Pharmacokinetic of Cabergoline by IMM route, and effects on udder engorgement and blood calcium concentrations at dry off in dairy cow.

10 lactating cows (9 Prim'holstein and 1 Brown Swiss) were included in this study. Cows were allocated to 2 groups:

| | |
|---|---|
| - Negative control group (n=5): | no treatment at D0 dry-off. |
| - Test group (n=5): | cabergoline IMM treatment at D0 dry-off, after last milking (1.34mg/quarter in all 4 quarters - same modalities as in example 1) |

| | | | |
|---|---|---|---|
| Animal characteristics were the following: | | control | cabergoline |
| | - Multiparous | parity 3 to 5 | parity 2 to 3 |
| | - Daily milk yield (L): | 17.2 +/- 1.3 | 17.6 +/-2.1 |
| | - Days in milk (d): | 242+/-47 | 229+/-38 |
| | - Body weight (kg) : | 728+/-31 | 689+/-69 |

No feed restriction was put in place within the 3 weeks preceding the D0 dry-off and after. Animal had free access to water and hay throughout the study. Each cow was given 7 kg/day of an antibiotic free concentrate feed for lactating cow until D0 dry off. After dry-off each cow was given 7kg/day of an antibiotic free concentrate for growing cattle. The composition of concentrate is given hereafter:

**Table 2**

| Feed name | Rumis L Pak (EVIALIS^{®}) | Patural Argent (EVIALIS^{®}) |
|---|---|---|
| | Concentrate feed for lactating cows | Concentrate feed for growing cattle |
| Crude protein | 18.0 % | 18.0 % |
| Crude cellulose | 13.0 % | 13.0 % |
| Crude ash | 5.9 % | 7.0 % |
| Crude fat | 4.5 % | 3.0 % |
| Sodium | 0.30 % | 0.28 % |
| Calcium | 0.7 % | 1.0% |
| Phosphorus | 0.65 % | 0.5 % |
| Milk Forage Units (UFL) | 0.95 | 0.90 |

At D0 dry-off, an abrupt milking cessation was performed.

The inter-teat perimeter (ITP) was considered as a proxy of udder volume and engorgement, and was measured before treatment udder, both before and after milking, and then evenly after treatment until 9 days post-treatment.

Blood was collected, and cabergoline and calcium concentrations were assessed at different time points: before treatment and until 6-days post-treatment with respect to cabergoline and until 3-days post-treatment with respect to calcium.

### 2.1 Effect on ITP (udder engorgement) :

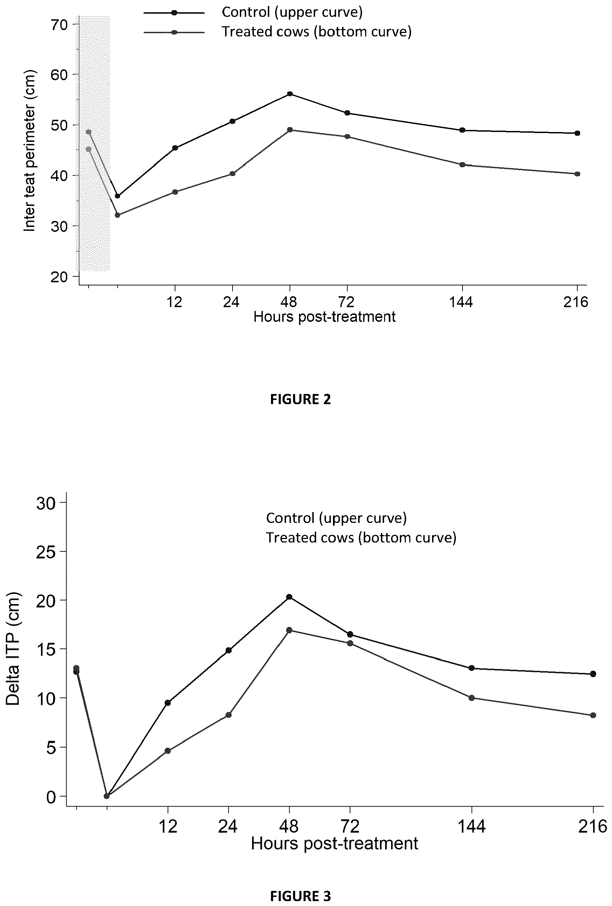

The ITP of treated cow increased less than control during the first 48 hours after treatment, and were always lower than the control during post dry-off follow-up, as illustrated in Figure 2.

The results are also demonstrative when expressed as the difference of ITP (Δ ITP), i.e. calculated by subtracting the ITP value after milking on D0 from ITP value at each time points after drying off, and as illustrated in Figure 3.

These delta ITP results obtained by IMM route are in the same range of efficacy, or somewhat better, as those by IM route.

In another study with the same total dose by IM route and under similar experimental conditions, delta ITP results obtained can be compared with the IMM results as follows:

**Table 3**

| Groups | | Before treatment Mean ΔITP before/after milking | After treatment Mean ΔITP at+24h |
|---|---|---|---|
| IMM study | Cabergoline (n=5) | 13.1 cm | 8.3 cm |
| | Control (n=5) | 12.7 cm | 14.8 cm |
| IM study | Cabergoline (n= 130) | 10.7 cm | 9.2 cm |
| | Control (n=133) | 11.4 cm | 15.7 cm |

### 2.2 Cabergoline blood concentrations

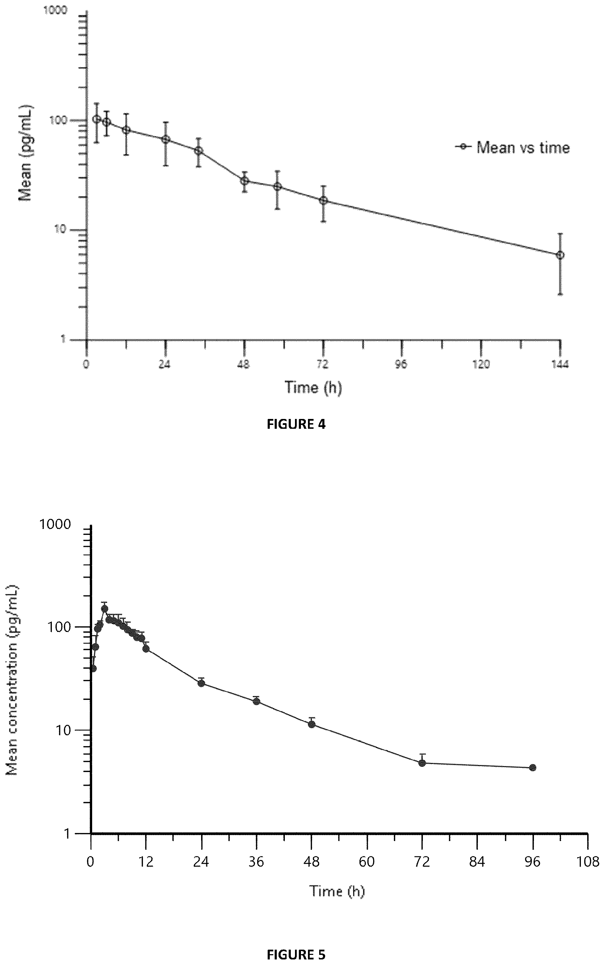

After Intra-mammary (IMM) administration of 5.4mg/udder of cabergoline, mean and sd plasma concentration - time profiles of cabergoline were as shown in Figure 4.

In a comparative study, cabergoline (Velactis^{™}, 1.12mg/ml) administered by intramuscular route at equivalent dose (5.6 mg/cow), showed the plasma concentration time-profiles depicted in Figure 5 (comparative study).

Pharmacokinetic parameters from both studies are compared in the following table:

**Table 4**

| PK parameters mean (sd) | IMM route (n=5) (sd) | IM route (n=6) (sd) |
|---|---|---|
| Cmax (pg/ml) | 114 (33) | 150 (22) |
| Tmax (h) | 8.4 (8.8) | 3.0 (0) |
| AUCo-inf (pg*h/ml) | **4746** (1423) | **2488** (257) |
| MRT (h) | 36.3 (7.1) | 22.7(3.6) |

| | | |
|---|---|---|
| AUCo-inf: area under the plasma drug concentration vs time curve from time zero to infinity; Cmax: observed maximum concentration of the drug Tmax: time at which Cmax is observed MRT: mean residence time of the drug in the plasma compartment Sd: standard deviation | | |

It can be concluded that at equivalent dose of 5.4 mg/cow (~7.5 µg/kg) the blood cabergoline bioavailability is higher by IMM route than by IM route, as assessed by a ratio AUC _{IMM route}/AUC _{IM route} around 1.9.

Interestingly, in the dose-effect study, as described in Example 1, AUCo-inf were the following:

**Table 5 (comparative)**

| *(Velactis*^{™}, *IM route oily solution 1.12mg*/*mL):* | |
|---|---|
| Doses tested (µg/kg) (N/group = 6) | AUCo-inf (pg*h/ml) Mean (sd) |
| 0 | / |
| 1.33 | 243 (83) |
| 4 | 1095(422) |
| 12 | 4999 (967) |

Together these PK results showed the good linear proportionality between cabergoline doses (ranging from 1.33 to 12 µg/kg) and their AUC, and consequently between AUC and cabergoline effect on milk production reduction. It also showed that a dose exposure at 7 to 8 µg/kg by IMM route is equivalent to dose exposure of a dose at 12µg/kg by IM route, and explained the better observed effect compared to 7 to 8µg/kg IM route.

It is concluded that IMM route represents a method to improve cabergoline efficacy compared to parenteral route (IM).

### 2.3 Blood calcium concentration

In comparison with control cows (i.e. without cabergoline treatment, see animal characteristics, example 2), treated cows by cabergoline IMM route exhibited only a slight decrease of blood calcium concentration (-0.2 mmol/L in mean) and limited at only +6 hours time point. No decrease was observed at time point before (+3h) and after (+12h, +24h and onward).

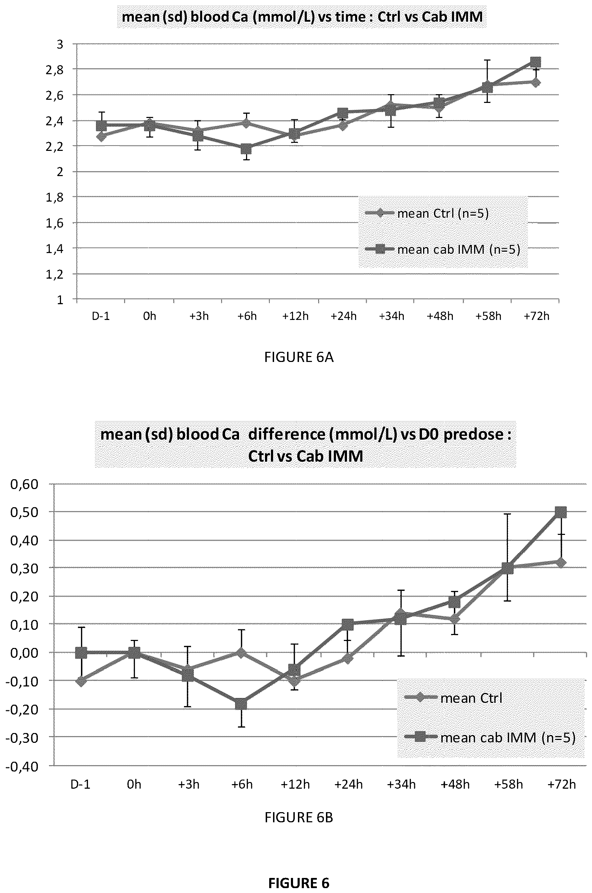

Figures 6 illustrate the time evolution of blood calcium concentrations in both groups. These results are surprising when compared to blood calcium concentration after IM route cabergoline treatment at the same total dosage (5.6 mg/cow), and besides IMM route exhibited a higher systemic exposure than IM route as explained above (ex. 2.2).

From studies performed previously using a dose of 5.6mg/cow cabergoline by IM route, a more important decrease of blood calcium was observed in time and extent. Said studies were conducted in comparison versus a negative control group, and blood calcium decrease was monitored and well identified: A calcium decrease was observed as soon as 2 to 3 hours post-treatment, maximal between 6 and 12 hours and return to normal after 24 hours.

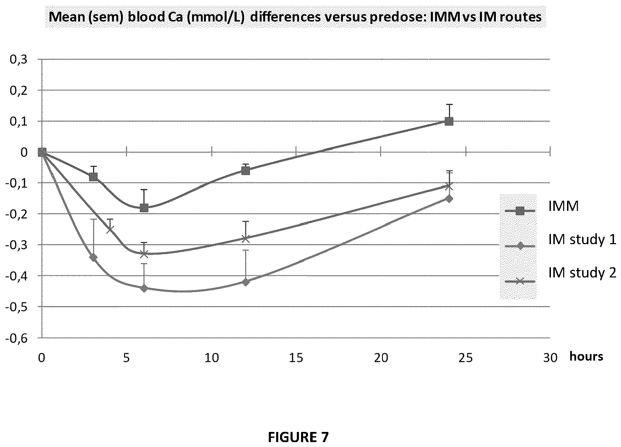

Figure 7 illustrates the difference of mean blood calcium relative to pre-treatment baseline values according time between the IMM study (top curve) as detailed above and IM studies (2 bottom curves, named IM studies 1 and 2 in the legend of Figure 7 ):
It is important to note that, in the study of example 2, the benefit regarding a less decrease of blood calcium concentration was obtained, despite a less quantity of calcium supplied by food, when compared to the IM studies 1 and 2. In these last 2 IM studies, 150 g of calcium/day/cow was provided through a total or partial mixed ration, whereas in the IMM study of example 2 only 50 g of calcium was provided via the concentrate (7 kg/day/cow at 0.7% of Ca) and a remaining part via the hay given ad libidum. Although the exact quantity of Ca provided by the hay was unknown, it was surely less than in the 2 IM studies, because,
i) the content of calcium in the hay is generally lower than concentrates, usually around 0.3%
ii) the actual hay dry matter intake could be estimated around 13 to 15 kg for the cows included in example 2

The quantity of calcium provided could be roughly estimated around 100 g/d/ cow in example 2, so clearly less than the 150 g of calcium as in the two IM studies (two bottom curves, named IM studies 1 and 2 in Figure 7).

Cabergoline use by IMM route has the surprising ability to less disturb calcium homeostasis in dairy cow compared to IM route at the same dosage, and even in a context of less stringent calcium recommendations to provide in the food.

Hence cabergoline use by IMM route has a better safety profile regarding cow recumbency's risk due to any hypocalcemia related to cabergoline treatment, meanwhile keeping the same or better efficacy when compared to IM route (according to the IMM dose chosen to be administered) for the drying-off of dairy cows.

In conclusion, the IMM route is a method to improve the safety and efficacy of cabergoline for the drying off in dairy cows in comparison to the known IM route for this indication. IMM cabergoline administration allows to reduce milk production more efficiently than by IM route, which is explained by a higher blood cabergoline bioavailability. IMM cabergoline administration also enables to prevent ruminants from hypocalcemia during the dry period, and more specifically just after drying-off (in particular within the first 24 hours following the last milking).

## Claims

1. A veterinary composition comprising cabergoline for use in a treatment that reduces milk production of a dairy ruminant, wherein said composition is administered into said dairy ruminant by intramammary route.

2. The veterinary composition for use according to claim 1, wherein the composition is administered at the time of the implementation of the dry period.

3. The veterinary composition for use according to claim 1, wherein the composition is administered at the time of the dry-off, after the last milking, preferably within the first 5, first 4, or first 2 hours after the last milking.

4. The veterinary composition for use according to any one of the preceding claims, wherein the therapeutic dose of cabergoline is between 1 and 50 µg/kg, or between 5 and 50 µg/kg, preferably about 5 to 11 µg/kg, and more preferably about 6 to 9 µg/kg.

5. The composition for use according to any one of the preceding claims, wherein the composition is administered in one or more quarters of the mammary gland by introduction of the composition via infusion, implantation, injection, or a combination thereof.

6. The composition for use according to any one of the preceding claims, wherein the composition is administered to all quarters of the mammary gland of the ruminant.

7. The composition for use according to any one of the preceding claims, wherein the amount of cabergoline which is intramammary administered is from 0.25 to 7 mg, preferably from 0.3 to 6 mg, and more specifically 1-6 mg, per dairy ruminant.

8. The composition for use according to any one of the preceding claims 1-6, wherein the amount of cabergoline which is intramammary administered is from 2 to 7 mg, preferably from 3 to 6 mg, and more specifically 5-6 mg, per dairy cow.

9. The composition for use according to any one of the preceding claims, **characterized in that** the ruminants are herbivorous mammals selected from bovines, ovines, caprines, camelids or bovids.

10. The composition for use according to any one of the preceding claims, wherein the ruminants are milk-producing ruminant mammals, preferably dairy cows, sheeps or goats, and more preferably dairy cows.

11. A kit for use in a treatment that reduces milk production of a dairy ruminant, wherein said kit includes at least one compartment for a packaging including an effective therapeutic amount of cabergoline, for intramammary administration to ruminants.

12. The kit for use according to claim 11, which also includes the means enabling the administration of the cabergoline composition by intramammary route and/or instructions relating to the mode and time of administration of the veterinary compositions.

## Patentansprüche

1. Veterinärzusammensetzung, umfassend Cabergolin zur Verwendung in einer Behandlung, die eine Milchproduktion eines Milchwiederkäuers reduziert, wobei die Zusammensetzung durch einen intramammären Weg in den Milchwiederkäuer verabreicht wird.

2. Veterinärzusammensetzung zur Verwendung nach Anspruch 1, wobei die Zusammensetzung zu dem Zeitpunkt der Implementierung der Trockenperiode verabreicht wird.

3. Veterinärzusammensetzung zur Verwendung nach Anspruch 1, wobei die Zusammensetzung zu dem Zeitpunkt des Trockenstellens nach dem letzten Melken, vorzugsweise innerhalb der ersten 5, ersten 4 oder ersten 2 Stunden nach dem letzten Melken verabreicht wird.

4. Veterinärzusammensetzung zur Verwendung nach einem der vorstehenden Ansprüche, wobei die therapeutische Dosis von Cabergolin zwischen 1 und 50 µg/kg oder zwischen 5 und 50 µg/kg, vorzugsweise etwa 5 bis 11 µg/kg und mehr bevorzugt etwa 6 bis 9 µg/kg beträgt.

5. Zusammensetzung zur Verwendung nach einem der vorstehenden Ansprüche, wobei die Zusammensetzung in einem oder mehreren Viertel der Mammadrüse durch Einführung der Zusammensetzung über Infusion, Implantation, Injektion oder einer Kombination davon verabreicht wird.

6. Zusammensetzung zur Verwendung nach einem der vorstehenden Ansprüche, wobei die Zusammensetzung allen Viertel der Mammadrüse des Wiederkäuers verabreicht wird.

7. Zusammensetzung zur Verwendung nach einem der vorstehenden Ansprüche, wobei die Menge an Cabergolin, die intramammär verabreicht wird, 0,25 bis 7 mg, vorzugsweise 0,3 bis 6 mg und spezieller 1-6 mg pro Milchwiederkäuer beträgt.

8. Zusammensetzung zur Verwendung nach einem der vorstehenden Ansprüche 1 bis 6, wobei die Menge an Cabergolin, die intramammär verabreicht wird, 2 bis 7 mg, vorzugsweise 3 bis 6 mg und spezieller 5-6 mg pro Milchkuh beträgt.

9. Zusammensetzung zur Verwendung nach einem der vorstehenden Ansprüche, **dadurch gekennzeichnet, dass** die Wiederkäuer herbivore Säugetiere sind, die aus Rindern, Schafen, Ziegen, Kameliden oder Boviden ausgewählt sind.

10. Zusammensetzung zur Verwendung nach einem der vorstehenden Ansprüche, wobei die Wiederkäuer milchproduzierende Wiederkäuer, vorzugsweise Milchkühe, Schafe oder Ziegen und mehr bevorzugt Milchkühe sind.

11. Kit zur Verwendung in einer Behandlung, die die Milchproduktion eines Milchwiederkäuers reduziert, wobei das Kit mindestens ein Fach für eine Verpackung, einschließlich einer wirksamen therapeutischen Menge von Cabergolin, für eine intramammäre Verabreichung an Wiederkäuer einschließt.

12. Kit zur Verwendung nach Anspruch 11, das auch die Mittel, die die Verabreichung der Cabergolinzusammensetzung durch den intramammären Weg ermöglichen und/oder Anweisungen bezüglich des Modus und der Zeit der Verabreichung der Veterinärzusammensetzungen einschließt.

## Revendications

1. Composition vétérinaire comprenant de la cabergoline pour utilisation dans un traitement qui réduit la production de lait d'un ruminant laitier, ladite composition étant administrée audit ruminant laitier par voie intramammaire.

2. Composition vétérinaire pour utilisation selon la revendication 1, la composition étant administrée au moment de la mise en oeuvre de la période de tarissement.

3. Composition vétérinaire pour utilisation selon la revendication 1, la composition étant administrée au moment du tarissement, après la dernière traite, de préférence au cours des 5 premières, des 4 premières, ou des 2 premières heures après la dernière traite.

4. Composition vétérinaire pour utilisation selon l'une quelconque des revendications précédentes, dans laquelle la dose thérapeutique de cabergoline est comprise entre 1 et 50 µg/kg, ou entre 5 et 50 µg/kg, de préférence d'environ 5 à 11 µg/kg, et plus préférablement d'environ 6 à 9 µg/kg.

5. Composition pour utilisation selon l'une quelconque des revendications précédentes, la composition étant administrée dans un ou plusieurs quartiers de la glande mammaire par introduction de la composition par l'intermédiaire d'une perfusion, implantation, injection ou d'une combinaison de celles-ci.

6. Composition pour utilisation selon l'une quelconque des revendications précédentes, la composition étant administrée à tous les quartiers de la glande mammaire du ruminant.

7. Composition pour utilisation selon l'une quelconque des revendications précédentes, dans laquelle la quantité de cabergoline qui est administrée par voie intramammaire va de 0,25 à 7 mg, de préférence de 0,3 à 6 mg, et plus spécifiquement 1 à 6 mg, par ruminant laitier.

8. Composition pour utilisation selon l'une quelconque des revendications 1 à 6 précédentes, dans laquelle la quantité de cabergoline qui est administrée par voie intramammaire va de 2 à 7 mg, de préférence de 3 à 6 mg, et plus spécifiquement 5 à 6 mg, par vache laitière.

9. Composition pour utilisation selon l'une quelconque des revendications précédentes, **caractérisée en ce que** les ruminants sont des mammifères herbivores choisis parmi bovins, ovins, caprins, camélidés ou bovidés.

10. Composition pour utilisation selon l'une quelconque des revendications précédentes, dans laquelle les ruminants sont des mammifères ruminants producteurs de lait, de préférence des vaches, moutons ou chèvres, laitiers, et plus particulièrement des vaches laitières.

11. Trousse pour utilisation dans un traitement qui réduit la production de lait d'un ruminant laitier, ladite trousse comportant au moins un compartiment pour un emballage comportant une quantité thérapeutique efficace de cabergoline, pour administration intramammaire à des ruminants.

12. Trousse pour utilisation selon la revendication 11, qui comporte également le moyen permettant l'administration de la composition de cabergoline par voie intramammaire et/ou des instructions se rapportant au mode et au moment d'administration de la composition vétérinaire.
